# EUROPEAN PATENT APPLICATION

(11) **EP 4 623 938 A1**
(43) Date of publication of application: **01.10.2025**
(21) Application number: 24315112.3
(22) Date of filing: 28.03.2024
(51) Int. Cl.: A61K 51/04

(54) **FLUORINE-18 COMPLEX USEFUL FOR POSITRON-EMISSION TOMOGRAPHY**

(71) Applicant: Conservatoire National des Arts et Métiers, 75003 Paris (FR); Université Paris Cité, 75006 Paris (FR)
(72) Inventor: Dioury, Fabienne, 91140 Villebon-sur-Yvette (FR); San, Carine, 93000 Bobigny (FR); Port, Marc, 95170 Deuil-la-Barre (FR); Sarda-Mantel, Laure, 92400 Courbevoie (FR)
(74) Representative: Vial, Lionel

(57) **Abstract**

The present invention relates to a complex of ¹⁸F and of a compound of the following formula (I): 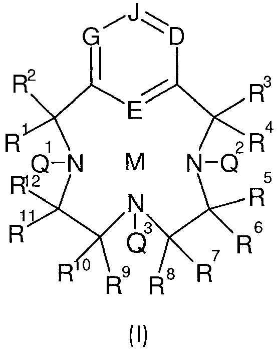 wherein ¹⁸F is coordinated to M.

## Description

### Field of the invention

The present invention relates to a fluorine-18 (¹⁸F) complex and its use in positron-emission tomography (PET) imaging methods.

### Technical background

Positron-emission tomography (PET) is a functional imaging technique that allows to probe biological processes *in vivo.* PET imaging has notably been used to successfully diagnose and monitor the progression of diseases.

PET is based on the detection of positron emission by radionuclides that have been administered as tracers to an individual to be imaged. The emitted positron travels a certain distance (positron range), depending on its energy, and undergoes inelastic collisions until the kinetic energy is so low that the timely overlap with an electron and annihilation is possible.

PET radionuclides are typically generated via nuclear reactions within a cyclotron or by specific decay mechanisms from a generator. The resulting radionuclides are incorporated through a variety of available radiochemical reactions into molecules, thus yielding the desired tracers. After having been administered the tracer, the individual is subsequently scanned to quantify the radioactive signal which provides information on the annihilation site, and thereby, the tracer localization at different organs.

Among the various existing positron-emitting nuclides fluorine-18 (¹⁸F) is the most widely used radionuclide for PET due to its unique advantages over other PET nuclides. These advantages include:
- a low positron range below 2.4 mm which allows the generation of high spatial resolution images,
- a clear positron emission profile comprising 97% positron emission and 3% electron capture (EC), and
- an optimal physical half-live of 109.8 min that enables off-site use in satellite facilities without a cyclotron.

Fluorine atom is typically not a constituent of most biomolecules and hence ¹⁸F is frequently introduced into tracers via creation of a C-F bond via nucleophilic substitution (Rong et al. (2023) Nature Communications 14:3257-3279).

However, nucleophilic substitution is generally a multi-step reaction which takes about an hour, thereby leading to a substantial loss of positron emission capacity of thus obtained ¹⁸F-containing tracers prior to their administration. Besides, this reaction is generally conducted at high temperatures and low pH which is disadvantageous when working with bioconjugates bearing thermally and pH sensitive biomolecules.

Accordingly, the need for methods to introduce fluorine-18 into biomolecules under mild conditions, ideally in a single step, has led to attempts to depart from conventional C-F bond formation as a basis for ¹⁸F bioconjugate synthesis, toward metal fluorine bonds (San et al. (2024) « Radiomarquages au fluor-18 : en quête de méthodes douces pour le développement de radiotraceurs TEP plus spécifiques» Actualité chimique)

In this regard, Gallium features prominently in the search for suitable metallic fluoride-binding sites, with a range of Ga(III) complexes with both macrocyclic and acyclic chelates being explored as alternatives. However, the use of metal complexes as binding sites for [¹⁸F]fluoride has not yet led to the ideal mild, aqueous one-step ¹⁸F labeling and/or stable ¹⁸F radiotracer (Blower et al. (2021) Advances in Inorganic Chemistry 78:1-35).

It is therefore an object of the present invention to provide metal complexes that can efficiently and rapidly bind to ¹⁸F under mild conditions, in particular at room temperature in a pH range adapted to avoid denaturation of biomolecule, and in predominantly aqueous conditions, to yield a stable ¹⁸F radiotracer in *vivo.*

### Summary of the invention

The present invention follows from the finding, by the present inventors, that complexes such as Ga¹⁸F-L¹ could efficiently and rapidly incorporate ¹⁸F under mild and aqueous conditions, thereby providing a stable ¹⁸F radiotracer in solution.

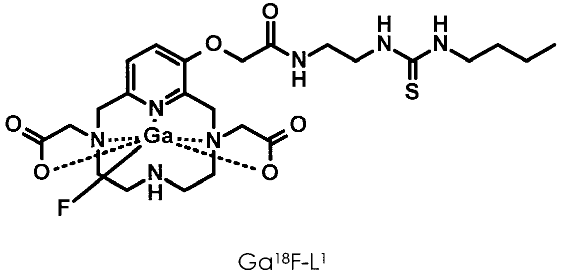

The present invention thus relates to a complex of F, in particular ¹⁸F, and of a compound of the following formula (I): 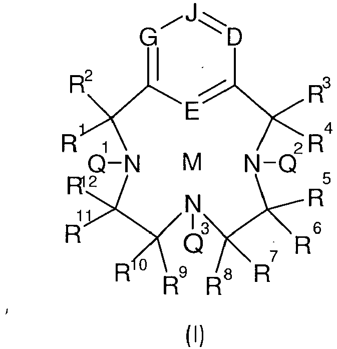 wherein:
- E represents CH, N or NO;
- M represents a cation selected from the list consisting of Al³⁺, Ga³⁺, In³⁺, Sc³⁺, Y³⁺, La³⁺, Ce³⁺, Pr³⁺, Nd³⁺, Sm³⁺, Eu³⁺, Gd³⁺, Tb³⁺, Dy³⁺, Ho³⁺, Er³⁺, Tm³⁺, Yb³⁺, Lu³⁺ and Fe³⁺, and is coordinated to at least one of the N atoms linked to Q¹, Q² and Q³ and to E when E represents N or NO;
- D, G and J, identical or different, represent a group selected from the list consisting of CH, N, C-OH, C-O-L and C-L, wherein L represents a linker group, optionally linked to a biological targeting moiety (BTM);
- R¹, R², R³, R⁴, R⁵, R⁶, R¹¹ and R¹², identical or different, represent a group selected from the list consisting of -H, -OH, -(CH₂)ₙ-CH₃, -(CH₂)ₙ-OH and -L, wherein n represents an integer from 0 to 5,
- R⁷, R⁸, R⁹ and R¹⁰, identical or different, represent a group selected from the list consisting of -H, =O, -(CH₂)ₚ-CH₃, -(CH₂)ₚ-OH and -L, wherein p represents an integer from 0 to 3, provided that when one of R⁷ or R⁸ and/or one of R⁹ or R¹⁰ represents =O then the other one is absent, i.e. represents no group;
- Q¹, Q² and Q³, identical or different, represent a group selected from the list consisting of -H, -OH, -(CH₂)ₖ-CH₃, -(CH₂)ₖ-OH, -CHQ⁴X and -L, wherein k represents an integer from 0 to 3, Q⁴ represents H or a C₁₋₃ alkyl group, and X represents a group selected from the list consisting of -COO-, -PO(OH)(O⁻) and -Q⁵-PO(OH), wherein Q⁵ represents Ar or a C₁₋₃ alkyl group, provided that at least two of Q¹, Q² and Q³ represent -CHQ⁴X and are coordinated to M; provided that the compound of formula (I) comprises at least one L,
wherein F, in particular ¹⁸F, is coordinated to M.

The present invention also relates to the complex as defined above wherein F is ¹⁸F and the compound is linked to a biological targeting moiety (BTM), for use as an *in vivo* PET scan diagnostic agent.

The present invention also relates to the use of a complex as defined above, wherein F is ¹⁸F and the compound is linked to a biological targeting moiety (BTM), for generating a PET scan image of a body or a body part of an individual, wherein the complex has been administered previously to the individual.

The present invention also relates to a method of imaging an individual, comprising generating an image using PET of a body or body part of the individual, to which a complex as defined above, wherein F is ¹⁸F and the compound is linked to a biological targeting moiety (BTM), has distributed, wherein the complex has been administered previously to the individual.

The present invention also relates to a pharmaceutical, imaging or diagnostic composition comprising a complex as defined above, wherein F is ¹⁸F and the compound is linked to a biological targeting moiety (BTM), optionally in association with at least one biocompatible carrier.

The present invention also relates method of preparation of the complex as defined above, wherein F is ¹⁸F, which comprises:
- reacting a compound of formula (I), wherein M is bound to R¹³, with a ¹⁸F fluoride anion, wherein R¹³ represents -OH, -OCH₃, -OCH₂-CH₃, -CO-CH₃, -CO-CH₂-CH₃, preferably -OH, or
- reacting a complex of ¹⁹F and a compound of formula (I) wherein ¹⁹F is coordinated to M, with a ¹⁸F fluoride anion, or
- reacting a complex of ¹⁸F and M, wherein M is as previously defined, with a compound of the following formula (I'): 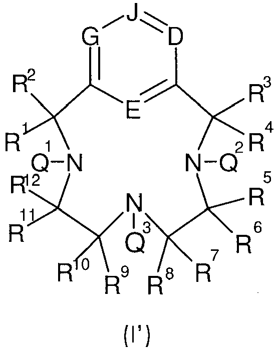
wherein D, E, G, J, Q¹, Q², Q³, R¹, R², R³, R⁴, R⁵, R⁶, R⁷, R⁸, R⁹, R¹⁰, R¹¹ and R¹² are as previously defined.

The present invention also relates to a kit or cassette comprising the non-radioactive reagents necessary to carry out the method as defined above, including:
- a compound of formula (I), wherein M is bound to R¹³, wherein R¹³ represents -OH, - OCH₃, -OCH₂CH₃, -CO-CH₃, -CO-CH₂-CH₃, preferably -OH, or
- a complex of ¹⁹F and a compound of formula (I) wherein ¹⁹F is coordinated to M, or
- a compound of formula (I').

The present invention also relates to a compound of formula (I) as defined above, wherein M is bound to R¹³, wherein R¹³ represents -OH, -OCH₃, -OCH₂-CH₃, -CO-CH₃, or -CO-CH₂-CH₃, preferably -OH.

The present invention also relates to a compound of formula (I).

The present invention also relates to a compound of formula (I').

### Description of the invention

### Definitions

As a preliminary remark, it should be noted that the term "consisting of" means "constituted by", i.e. when an object "consists of" an element or several elements, the object cannot include other elements than those mentioned. In contrast, the term "comprising" means "including", "containing" or "encompassing", *i.e.* when an object "comprises" an element or elements, other elements than those mentioned can also be included in the object. In other words, when an object "comprises" an element or elements, it consists of the element(s) and possibly of other elements than these.

As should be clear to a person of skill in the art, for clarity reasons the coordinate bonds involving M and neighbouring doublet carrying atoms, such the N atoms linked to Q¹, Q² and Q³, E when E represents N or NO as well as Q¹, Q² and Q³, are not represented in the formulae shown herein, except in the Examples where the coordinate bonds are represented.

As intended herein, "Ar" represents a C5-12 arylene group or a C3-12 heteroarylene group.

As intended herein, the expression "biological targeting moiety" (BTM) relates to a compound which, after administration, is taken up selectively or localises at a particular site of the mammalian body *in vivo.* Such sites may for example be implicated in a particular disease state or be indicative of how an organ or metabolic process is functioning.

As intended herein, the BTM according to the invention includes pre-targeting moieties. The so-called pre-targeting strategy is well known to the person of skill in the art. It is notably described in Verhoeven et *al*. (2019) Pharmaceutics **11(9)**:434. According to this strategy a non-radioactive biological targeting compound, such as. an antibody, which is taken up selectively or localises at a particular site of the mammalian body *in vivo* is first administered. A radioactive tracer comprising a pre-targeting moiety which can bind to the biological targeting compound *in vivo* is then administered. The advantage of the strategy lies in the fact that the biological targeting compound being non-radioactive it is possible to wait the necessary time for it to accumulate in sufficient amount at the targeted site. The expression "pre-targeting moiety" thus relates to a moiety which can bind to a biological targeting compound *in vivo.*

### Compound of formula (I) or (I')

Preferably, the above-defined compound of formula (I) is of the following formula (I-1): 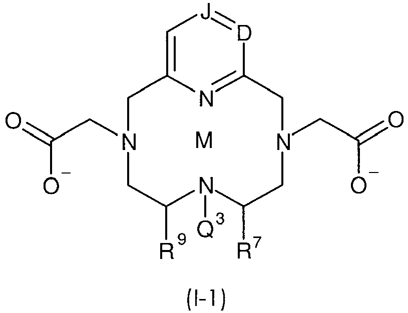 wherein:
- R⁷ and R⁹, identical or different, represents =O or -H, and
- D represents C-O-L, J represents C-H, and Q³ represents -H, -OH, -(CH₂)ₖ-CH₃, -(CH₂)ₖ-OH wherein k represents an integer from 0 to 3, or
- D represents C-H, J represents C-L or C-O-L, and Q³ represents -H, -OH, -(CH₂)ₖ-CH₃, - (CH₂)ₖ-OH wherein k represents an integer from 0 to 3, or
- D represents C-H, J represents C-H, and Q³ represents L,
wherein L is as defined above.

Preferably, the above-defined compound of formula (I') is of the following formula (I-1'): 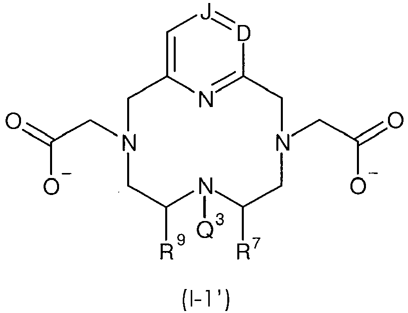 wherein:
- R⁷ and R⁹, identical or different, represents =O or -H, and
- D represents C-O-L, J represents C-H, and Q³ represents -H, -OH, -(CH₂)ₖ-CH₃, -(CH₂)ₖ-OH wherein k represents an integer from 0 to 3, or
- D represents C-H, J represents C-L or C-O-L, and Q³ represents -H, -OH, -(CH₂)ₖ-CH₃, - (CH₂)ₖ-OH wherein k represents an integer from 0 to 3, or
- D represents C-H, J represents C-H, and Q³ represents L,
wherein L is as defined above.

Preferably, in the above formulae (I), (I'), (I-1) and (I-1'), L is a group of formula -(A)ₘ-Y wherein
- Y represents a group selected from the list consisting of -H, -N=C=S, -N=C=O, - NH(C=S)-BTM, and -NH(C=O)-BTM,
- each A is independently selected from the list consisting of -CR₂-, -CR=CR-, -C≡C-, - CR₂CO₂-, -CO₂CR₂-, -NRCO-, -CONR-, -CONHCR₂NH(C=S)NH-, -CONHCR₂NH(C=O)NH- , -CR=N-O-, -NR(C=O)NR-, -NR(C=S)NR-, -SO₂NR-, -NRSO₂-, -CR₂OCR₂-, -CR₂SCR₂-, - CR₂NRCR₂-, -CR₂NH(C=S)NH-, -CR₂NH(C=O)NH-, a C₄₋₈ cycloheteroalkylene group, a C₄₋₈ cycloalkylene group, -Ar-, -NR-Ar-, -O-Ar-, -Ar-(CO)-, an amino acid, a sugar or a polyethyleneglycol (PEG) building block,
wherein:
- each R is independently selected from the list consisting of H, C₁₋₆ alkyl, C₂₋₄ alkenyl, C₂₋₄ alkynyl, C₁₋₄ alkoxyalkyl, C₁₋₄ hydroxyalkyl, Ar-NH(C=S)NH, Ar-NH(C=O)NH, C₁₋₄ alkylphenyl-NH(C=S)NH, C₁₋₄ alkylphenyl-NH(C=O)NH,
- m is an integer from 1 to 20,
- each Ar is independently a C₅₋₁₂ arylene group, or a C₃₋₁₂ heteroarylene group.

Preferably, in the above formulae (I), (I'), (I-1) and (I-1'), L is selected from the list consisting of:

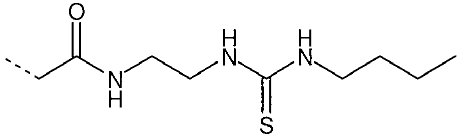

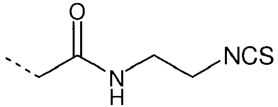

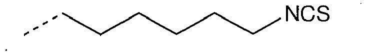

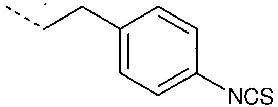

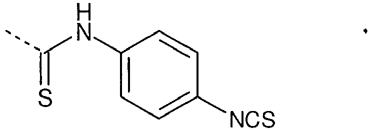

As should be clear to the person skilled in the art -NCS represents -N=C=S

Preferably, M represents Ga³⁺.

Preferably, the compound of formula (I) is represented by a formula selected from the list consisting of:

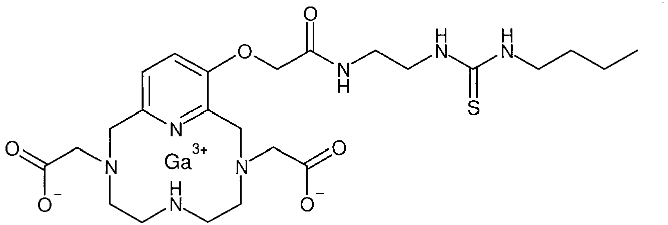

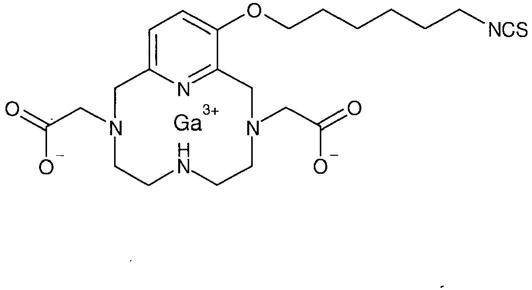

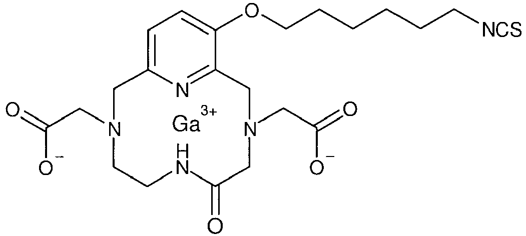

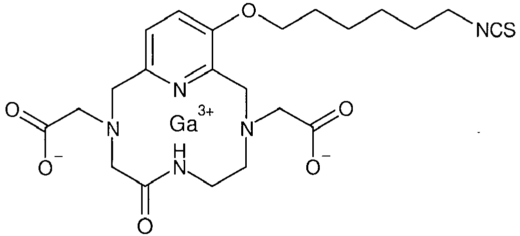

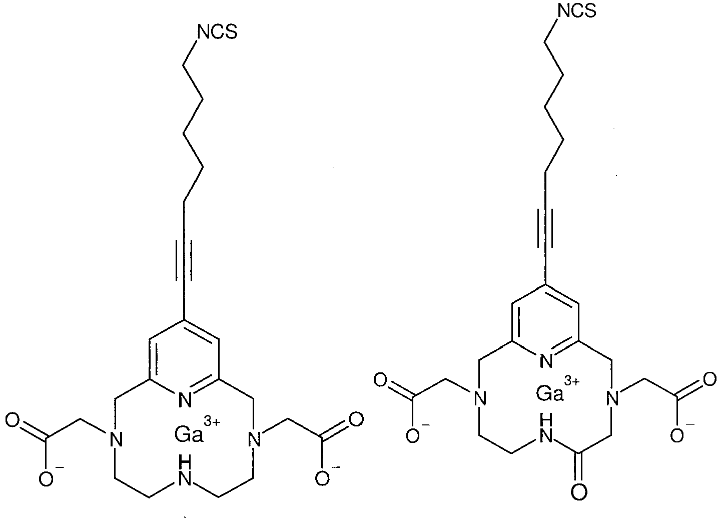

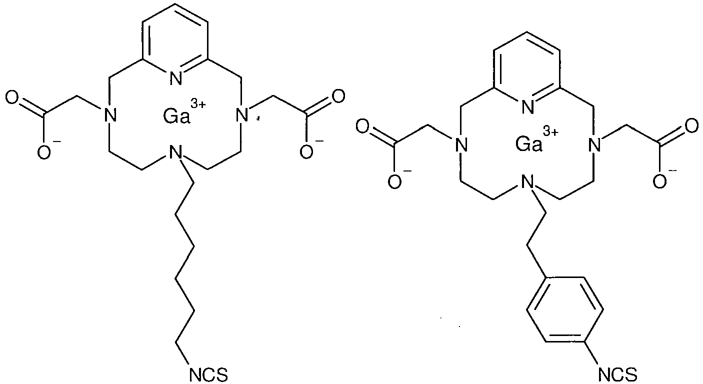

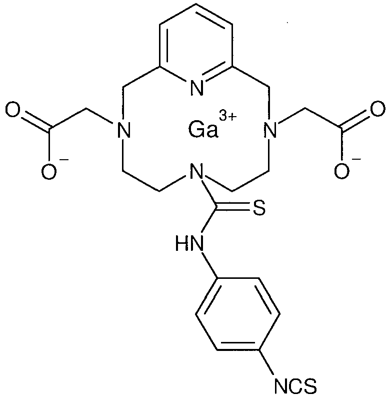

Preferably, the compound of formula (I) or (I') according to the invention is linked to a biological targeting moiety (BTM).

Preferably, the biological targeting moiety (BTM) is selected from the list consisting of a pre-targeting moiety, an amino-acid, a polypeptide, in particular comprising from 3 to 100 amino-acid residues, a peptoid or peptidic mimetic, an enzyme substrate, agonist or inhibitor, a ligand of a biological receptor, an antibody, an antibody fragment, a scFv, a VHH and an aptamer.

By way of example, the pre-targeting moiety can be avidin, streptavidin, biotin, a single-stranded nucleic acid, an antibody, an antibody fragment, a scFv, a VHH, an aptamer, a click-chemistry unit, such as trans-cyclooctene or tetrazine.

In an embodiment of the invention, in the compound of formula (I) according to the invention, M is bound to R¹³, wherein R¹³ represents -OH, -OCH₃, -OCH₂-CH₃, -CO-CH₃, or -CO-CH₂-CH₃. In that case, the compound of formula (I) of the invention wherein M is bound to R¹³ is useful as a non-radioactive precursor or synthesis intermediate which can yield the complex of the invention wherein F is ¹⁸F by ionic exchange.

### Complex

In a preferred embodiment of the invention, in the compound of formula (I), M is coordinated to ¹⁸F.

As will be clear to the person of skill in the art, the complex of the invention wherein F is ¹⁸F is useful as a tracer in PET imaging methods, *i.e.* as a PET agent.

In another embodiment of the invention, in the compound of formula (I), M is coordinated to ¹⁹F.

In that case, the complex of the invention wherein F is ¹⁹F is useful as a non-radioactive precursor or synthesis intermediate which can yield the complex of the invention wherein F is ¹⁸F by isotopic exchange.

### Use - Method of imaging

As should be clear to the person of skill in the art, in the use or the method of imaging according to the invention, the step of administering the complex according to the invention or a composition comprising it to the individual is not part of the use or the method of imaging of the invention. It has been administered before implementing the use or the method of imaging according to the invention. As such, the use or the method of imaging of the invention does not comprise a step of treatment of the human or animal body by surgery.

Besides, as should also be clear to the person of skill in the art, the use or the method of imaging according to the invention does not comprise a step of diagnosing a disease. A s such, the use or the method of imaging of the invention is not a diagnostic method practiced on the human or animal body.

The individual according to the invention can be human or an animal, in particular a mammal.

Preferably, the complex according to the invention is included in a pharmaceutical, imaging or diagnostic composition.

### Pharmaceutical, imaging or diagnostic composition

As should be clear to the person of skill in the art the pharmaceutical, imaging or diagnostic composition is in a form suitable for human or animal administration.

By the phrase "in a form suitable for human or animal administration" is meant a composition which is sterile, pyrogen- free, lacks compounds which produce toxic or adverse effects, and is formulated at a biocompatible pH (approximately pH 4.0 to 10.5). Such compositions also contain only biologically compatible excipients, and are preferably isotonic.

As intended herein, a "biocompatible carrier" is a fluid, especially a liquid, in which the imaging agent can be suspended or preferably dissolved, such that the composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is isotonic); an aqueous buffer solution comprising a biocompatible buffering agent; an aqueous solution of one or more tonicity-adjusting substances (*e.g.* salts of plasma cations with biocompatible counterions), sugars (*e.g.* glucose or sucrose), sugar alcohols (*e.g.* sorbitol or mannitol), glycols (*e.g.* glycerol), or other non-ionic polyol materials (*e.g.* poly ethyleneglycols, propylene glycols and the like). Preferably the biocompatible carrier is pyrogen-free water for injection, isotonic saline or phosphate buffer.

The pharmaceutical, imaging or diagnostic composition according to the invention may contain additional optional excipients such as: an antimicrobial preservative, pH-adjusting agent, filler, radioprotectant, solubilizer or osmolality adjusting agent. By the term "radioprotectant" is meant a compound which inhibits degradation reactions, such as redox processes, by trapping highly reactive free radicals, such as oxygen-containing free radicals arising from the radiolysis of water. The radioprotectants of the present invention may notably be suitably chosen from ascorbic acid, 4-aminibenzoic acid, gentisic acid and salts thereof with a biocompatible cation.

### Method of preparation, kit and cassette

In the method of preparation according to the invention the ¹⁸F fluoride anion can be provided under any form liable to yield a fluoride anion in the reaction mixture. In particular, the ¹⁸F fluoride anion can be provided in the form of a ¹⁸F fluoride salt, more particularly a salt of an alkaline metal. Preferably, the ¹⁸F fluoride anion is provided or added as Na¹⁸F.

As should be clear to the person of skill in the art, the compound of formula (I) wherein M is bound to R¹³, the complex of ¹⁹F and a compound of formula (I) or the compound of formula (I') is a precursor or a synthesis intermediate of the complex according to the invention wherein F is ¹⁸F.

Preferably, the method of preparation according to the invention is carried out using an automated synthesizer.

As intended herein, the expression "automated synthesizer" relates an automated module based on the principle of unit operations as notably described by Satyamurthy et al (1999) Clin. Positr. Imag. 2(5):233-253. The expression "unit operations" means that complex processes are reduced to a series of simple operations or reactions, which can be applied to a range of materials. Automated synthesizers are commercially available from a range of suppliers as notably mentioned by Satyamurthy *et al*. *op. cit.*

The automated synthesizer preferably comprises a cassette.

By the term "cassette" is meant a piece of apparatus designed to fit removably and interchangeably onto an automated synthesize, in such a way that mechanical movement of moving parts of the synthesizer controls the operation of the cassette from outside the cassette, i.e. externally. The cassette preferably comprises all the reagents, reaction vessels and apparatus necessary to carry out the preparation of a batch of a complex according to the invention wherein F is ¹⁸F.

Preferably, the precursor, the kit or cassette according to the invention is provided in sterile and/or lyophilized form. As should be clear to the person of skill in the art the precursor, kit or cassette is preferably non-radioactive.

The invention will be further described by the following non-limiting figures and examples.

### Description of the figures

Figure 1 and figure 2 show the stability of **Ga¹⁸F(L)** obtained with method 3 of Example 8. Figure 1: crude reaction mixture at 20 min ("*t₀*"); Figure 2: at 60 min.
Figure 3 and figure 4 represent the LC-UV-MS analysis (Figure 3: UV chromatogram, Figure 4: MS spectra at t_{R} = 5.6 - 6.0 min) of the decayed radiofluorination assay from method 3 of Example 8; crude reaction mixture analysed after 13 days at room temperature.

### EXAMPLES

### General procedures

### Synthesis of the macrocyclic pyclen derivatives

### • Synthesis of the triamine synthon 3.

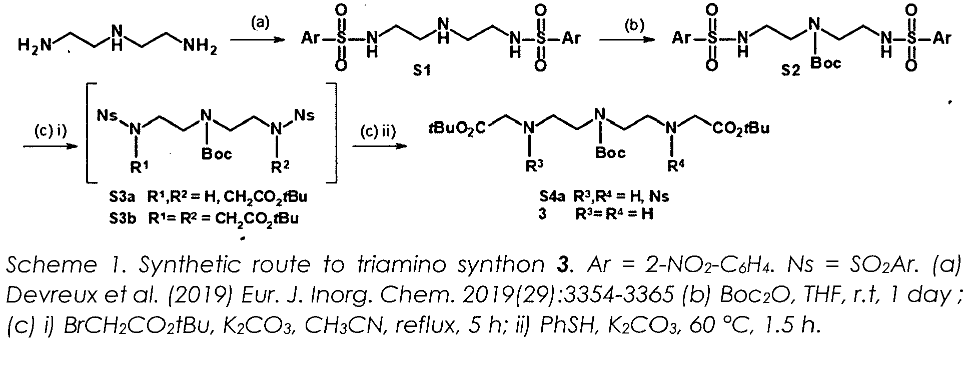

2-Nitro[N(2{2[(2-nitrophenyl)sulfonylamino]ethylamino}) ethyl] benzenesulfonamide **S1** was prepared as previously reported (Devreux et al. (2019) Eur. J. Inorg. Chem. 2019(29):3354-3365).

*tert*-Butyl bis[2-(2-nitrophenylsulfonamido)ethyl]carbamate **S2** was prepared according to reported protocols with slight modifications (Siaugue et al. (2001) Tetrahedron 57(22):4713-4718). Disulfonamido compound **S1** (19.87 g, 41.97 mmol) was suspended in tetrahydrofuran (115 mL) and di-tert-butyl dicarbonate was added portion wise (10.08 g, 46.17 mmol, 1.1 eq.). The reaction was stirred 1 day at room temperature (r.t.). The mixture was concentrated to dryness. The resulting crude oily material was purified by chromatography on silica gel (cyclohexane/AcOEt 5/5 to 4/6 v/v) and led to the desired product **S2** as a yellow oil that crystallized on standing (21.49 g, 37.47 mmol). Yield 89%. ¹H NMR (CDCl₃) *δ* (ppm): conform to reported data in CDCl₃ or DMSO-d6 (Siaugue et al. (2001) Tetrahedron 57(22):4713-4718; Ikeda et al. (2021) Bioorg. Med. Chem. Letters 32:127713).

*tert*-Butyl 2-[2-(*tert*-butoxycarbonyl-{2-[(2-*tert*-butoxy-2-oxoethyl)amino]ethyl}amino)ethylamino] acetate **3** was prepared according to reported protocols with slight modifications (Devreux et al. (2021) Inorg. Chem. 60(6):3604-3619). A suspension of the previously prepared disulfamido compound **S2** (16.76g, 29.21 mmol) with potassium carbonate (10.10g, 73.1 mmol, 2.5 eq.) in acetonitrile (175 mL) was refluxed for 15 minutes. *Tert*-Butyl bromoacetate (11 mL, 75.0 mmol, 2.5 eq.) was added in one portion. The reaction was monitored by mass spectrometry and/or TLC (cyclohexane/AcOEt 1/1 *v*/*v,* UV detection). After 5 hours, total conversion of both the starting amino compound **S2,** and of the mono-N-alkylated intermediate **S3a** was observed leading to the di-*N*-alkylated disulfonamide **S3b.** The reaction mixture was cooled, and a second portion of potassium carbonate (12.12 g, 87.7 mmol, 3.0 eq.) was added, followed by thiophenol (8.7 mL, 85.0 mmol, 2.9 eq.) in one portion. The reaction was monitored by mass spectrometry and/or TLC (cyclohexane/AcOEt 1/1 *v*/*v*, UV detection; CH₂Cl₂/MeOH 97.5/2.5 *v*/*v*, UV and ninhydrin stain detection). After 1.5 hours, total conversion of the intermediate compounds **S3b** and **S4a** was obtained. Warming was stopped and the reaction mixture was maintained in the hot bath that cooled to room temperature overnight. Insoluble matter was filtered off through a Celite^{®} pad, and the organic filtrate was concentrated to dryness. The resulting canary yellow colored oil was purified by chromatography on silica gel (CH₂Cl₂/MeOH 100/0 to 95/5 *v*/*v*) and led to the desired product **3** as a yellow oil that crystallizes in the freezer (12.12 g, 28.1 mmol). Yield 96%.

### • Synthesis of dibromide synthons

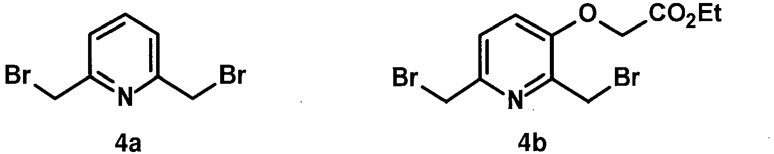

### Dibromide synthons prepared herein.

2,6-Bis(bromomethyl)pyridine **4a** was synthesized according to the protocol of Dioury et al. (2009) Tetrahedron 65(36):7573-7579.

Ethyl 2-[{2,6-bis(bromomethyl)pyridin-3-yl}oxy]acetate **4b** was prepared according to the previously reported protocol by Devreux et al. (2019) Eur. J. Inorg. Chem. 2019(29):3354-3365.

### • Macrocyclisation: general procedure

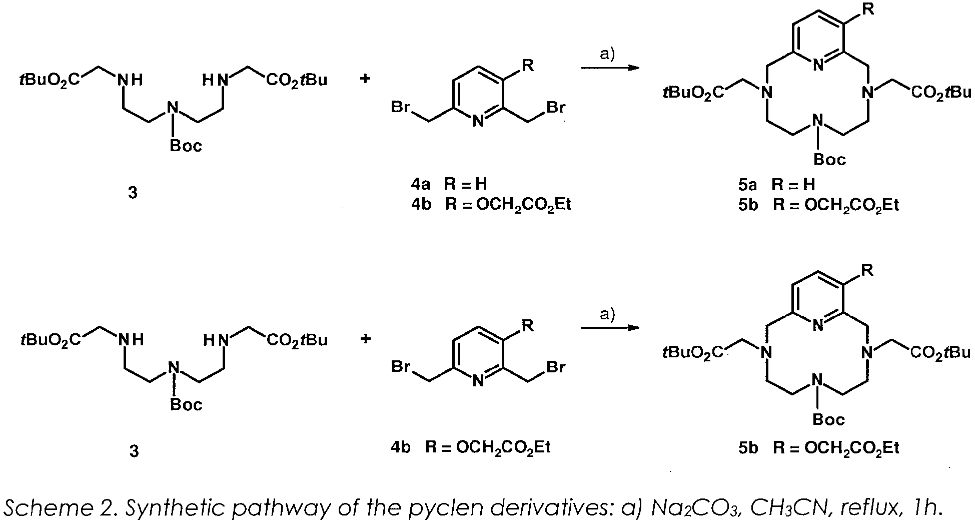

The dibromide **4b** (1.1-1.2 eq.) was added in one portion to a stirred suspension of the triamino synthon **3** with Na₂CO₃ (4 eq.) in refluxing acetonitrile (0.01 *M*). The reaction was monitored by mass spectrometry and/or TLC. After completion of the reaction, the carbonate was filtered off, and the clear filtrate was concentrated under reduced pressure.

### • Post-macrocycling functionalizations

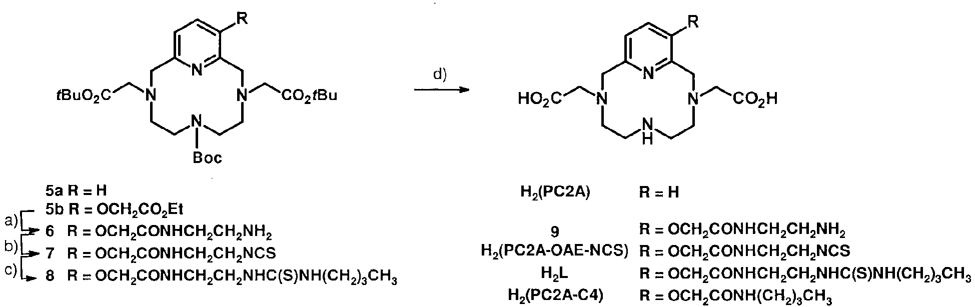

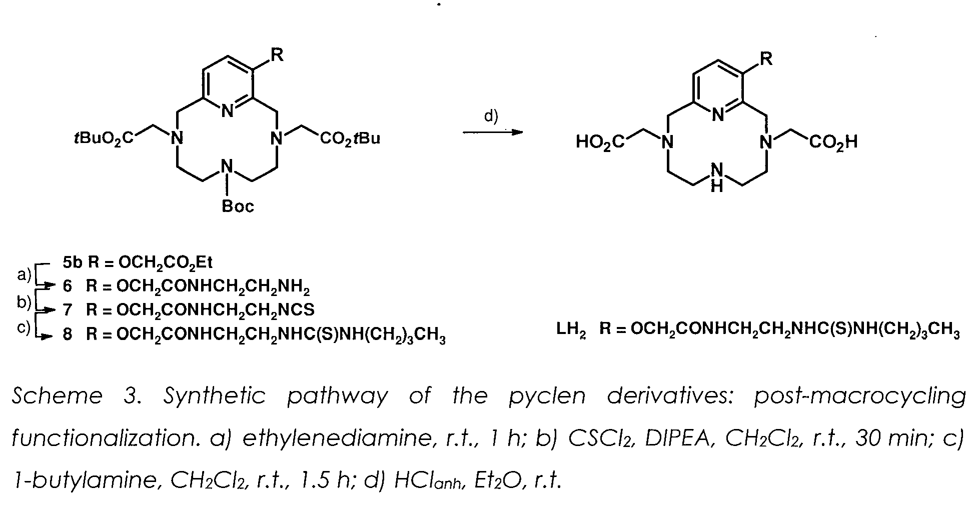

### Example 1

### Synthesis of tert-Butyl 3,9-bis(2-tert-butoxy-2-oxoethyl)-12-(2-ethoxy-2-oxo-ethoxy)-3,6,9,15tetraazabicycl o[9.3.1]pentadeca-1(15),11,13-triene-6-carboxylate 5b (Scheme 3. R = OCH₂CO₂Et).

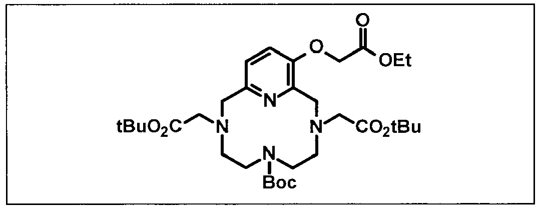

Formula: **C₃₂H₅₂N₄O₉**
Mwt: 636.78 g/mol
Monoisotopic Mwt: 650.400299

Done with pyridinic dibromide **4b** (467 mg, 1.27 mmol, 1.1 eq.; prepared according to the reported protocol by Devreux et al. (2019) Eur. J. Inorg. Chem. 2019(29):3354-3365), triamine **3** (500 mg, 1.16 mmol), and Na₂CO₃ (500 mg, 4.73 mmol, 4 eq.) in acetonitrile (120 mL); refluxed 1 hour. Macrocyclic compound **5b** was obtained as a yellow oil (864 mg) and was used as such in the next aminolysis step.

### Example 2

### Synthesis of tert-Butyl 12-[2-(2-amino-ethylamino)-2-oxo-ethoxy]-3,9-bis(2-tert-butoxy-2-oxoethyl)-3,6,9,15-tetrazabicyclo[9.3.1]pentadeca-1(15),11,13-triene-6-carboxylate 6.

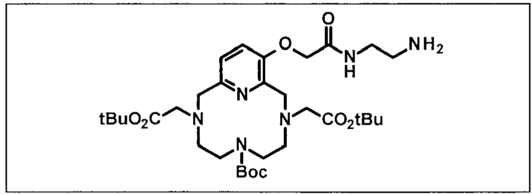

Formula: **C₃₂H₅₄N₆O₈**
Mwt: 650.81 g/mol
Monoisotopic Mwt: 650.400299

Done with the previously prepared macrocycle **5b** (848 mg, 1.16 mmol), ethylenediamine (3.4 mL, 51.72 mmol, 45 eq.); stirred at room temperature for 1 hour. The solution was diluted in dichloromethane (25 mL) and washed with water (1 × 5 mL). Purification was done by chromatography on silica gel (CH₂Cl₂/MeOH/NH₃ *7N* in MeOH 100/0/0 to 90/5/5).

Macrocyclic compound **6** obtained as a yellow solid (547 mg, 0.841 mmol, 73%).

### Example 3

### Synthesis of tert-Butyl 3,9-bis(2-tert-butoxy-2-oxoethyl)-12-{[(2-isothiocyanatoethyl)carbamoyl]methoxy}-3,6,9,15-tetraazabicyclo[9.3.1]pentadeca-1(15),11,13-triene-6-carboxylate 7.

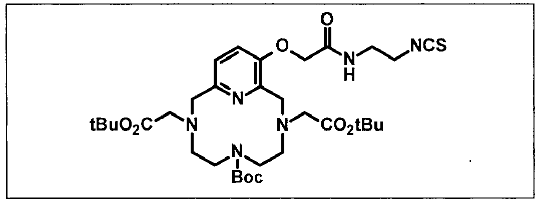

Formula: **C₃₃H₅₂N₆O₈S**
Mwt: 692.87 g/mol
Monoisotopic Mwt: 692.356714

To the previously prepared macrocycle **6** (1.031g, 1.58 mmol) solubilized in dichloromethane (30 mL), diisoproylethylamine (DIPEA, 825 µL, 4.85 mmol, 3 eq.) was added. A 0.4 *M* solution of thiophosgene (5.95 mL, 2.40 mmol, 1.5 eq.) in dried dichloromethane was then added, and the reaction mixture was stirred at room temperature. The reaction was monitored by TLC. After 30 min, completion of the reaction was reached. The solution was concentrated under reduced pressure. The crude residue was purified by flash chromatography on silica gel (100% AcOEt) to provide the desired product **7** (874 mg, 1.26 mmol, 80%) as a brown solid.

### Example 4

### Synthesis of tert-butyl 3,9-bis(2-tert-butoxy-2-oxo-ethyl)-12-[2-[2-(butylcarbamothioylamino)ethylamino]-2-oxo-ethoxy]-3,6,9,15-tetrazabicyclo[9.3.1]pentadeca-1(15),11,13-triene-6-carboxylate 8.

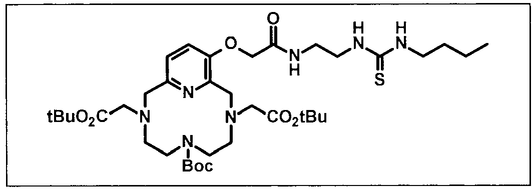

Formula: **C₃₇H₆₃N₇O₈S**
Mwt: 766.00 g/mol
Monoisotopic Mwt: 765.445948

To the previously prepared macrocycle **7** (240 mg, 0.346 mmol) solubilized in dichloromethane (5 mL), 1-butylamine (85 µL, 0.860 mmol, 2.5 eq.) was added and the solution was stirred at room temperature. The reaction was monitored by TLC. After 1h30, completion of the reaction was reached. The solution was concentrated under reduced pressure. The crude residue was purified by flash chromatography on silica gel (CH₂Cl₂/MeOH 100/0 to 95/5 *v*/*v*) to provide the desired product **8** (262 mg, 0.347 mmol, 99%) as an orange solid.

### Example 5

### Synthesis of 2-[12-[2-[2-(Butylcarbamothioylamino)ethylamino]-2-oxo-ethoxy]-9-(carboxymethyl)-3,6,9,15-tetrazabicyclo[9.3.1]pentadeca-1(14),11(15),12-trien-3-yl]acetic acid 13 (LH₂)

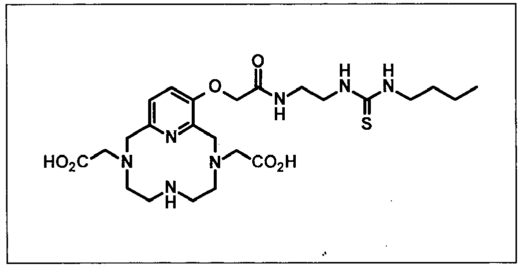

Formula: **C₂₄H₃₉N₇O₆S**
At pH 6.0, C₂₄H₃₃N₇NaO₆S / C₂₄H₃₉N₇O₆S / C₂₄H₄₀ClN₇O₆S **in ratio 79.1/20.1/0.7;** calculated at *pH* 6.0
Mwt: 571.32 g/mol (estimated at pH 6.0)

To the previously prepared macrocycle **8** (225 mg, 0.294 mmol) solubilized in dichloromethane (1 mL), a 2M solution of anhydrous HCl in Et₂O (21 mL, 42 mmol, 143 eq.) was added in one-portion under vigorous stirring at room temperature. The resulting heterogeneous medium was stirred until completion of the reaction (mass spectrometry monitoring; at least one day). The crude residue was alkalinized from *pH* 0.9 to *pH* 6.0 with NaOH_{aq} then purified by flash chromatography on RP18 silica gel (water/MeCN 90/10 *v*/*v*). Macrocyclic compound **LH₂** obtained at *pH* ≈ 6 as a white powder (105 mg, 0.184 mmol, 63%).

### Example 6

### Synthesis of {GaF} reagent "Na₂GaF₅"

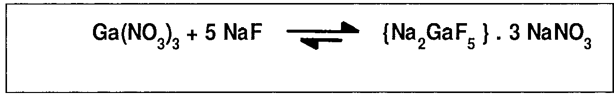

Formula: **F₅Ga.N₃Na₅O₉**
Mwt: 465.70 g/mol

The preparation of the water-soluble {GaF} reagent of formula referenced **"Na₂GaF₅"** was carried out according to the protocol described by Xu et al. (US pat. appl. pub. No. 2020/0390104). Sodium fluoride NaF (213 mg, 5.073 mmol, 5 eq.) was solubilized in ultrapure water (100 mL) giving a solution of *pH* 7.3. An aqueous solution of 0.0106 *M* (ICP evaluation) gallium nitrate (100 mL, 1.06 mmol) was added to the previously prepared solution of NaF and the resulting mixture was stirred at room temperature for 2 hours leading to a clear solution of *pH* 4.6 which gallium concentration was estimated by ICP titration: 4.76 *mM.* The resulting lyophilisate was diluted in water to different concentrations; interestingly, concentrated solutions (0.12 ou 0.06 *M*) led to a turbid liquid phase.

### Example 7

### Synthesis of gallium complexes GaX(L) with X = OH, F

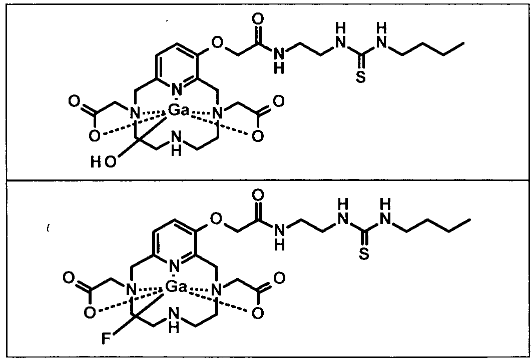

Formula: **C₂₄H₃₈GaN₇O₇S**
Mwt: 638.39 g/mol
Monoisotopic Mwt: 637.180933
Formula: **C₂₄H₃₇FGaN₇O₆S**
Mwt: 640.39 g/mol
Monoisotopic Mwt: 639.176600

For X = OH and/or F.

Protocol 1: An aqueous solution of 0.0529 *M* (ICP evaluation) **gallium nitrate** (5.2 mL, 0.275 mmol, 1.1 eq.) was added to the previously prepared **LH₂** ligand isolated at *pH* 6 (143 mg, 0.248 mmol based on anhydrous mono-sodium form). The pH of the reaction solution dropped to *pH* 1.4. A 1 *M* aqueous solution of NaF (*pH* 10.6) was gradually added to adjust the *pH* to 4.4 (3.1 mL, 3.1 mmol, 12.5 eq.). The reaction was monitored by mass spectrometry and proved complete after less than 5 minutes at room temperature. The reaction solution was concentrated to dryness. The desired product was obtained after removing several inorganic sodium salts introduced or formed during the process (NaCl, NaF, NaNO₃) by reverse phase chromatography on cyano(propyl)-modified silica gel (water/acetone 9/1 *v*/*v*) leading to the desired compound isolated by freeze-drying as a white solid (63 mg, 0.098 mmol based on **GaF(L)** anhydrous form, 40%).

Protocol 2: A solution of the previously prepared ligand **LH₂** (28 mg, 45.8 pmol) in ultrapure water (3.7 mL) was adjusted to *pH* 5.3 with an aqueous solution of NaOH. An aqueous solution of reagent **"Na₂GaF₅"** previously prepared and titrated by ICP (5.61 mM at *pH* 4.6, 8.5 mL, 47.7 pmol, 1 eq.) was added dropwise under magnetic stirring at room temperature for 5 minutes. The *pH* dropped to *pH* 3.8 and was carefully adjusted to *pH* 5.1 with an aqueous solution of NaOH (0.1 *M*, 50 µL, 50 µmol, 1.1 eq.). A monitoring by mass spectrometry indicated a total conversion to the desired gallium complex. The resulting mixture was concentrated by freeze-drying to give a pale pink solid (90 mg vs ca. 30 mg anhydrous form of expected compound) corresponding to a mixture of the desired gallium complex with several inorganic sodium salts introduced or formed during the process (NaCl, NaF, NaNO₃). Interestingly, ¹H NMR analysis of the crude material revealed the presence of glycerol, which might have originated from various plastic devices involved in the process. An aliquot of crude compound (60 mg) was purified by reverse phase HPTLC on cyano(propyl)-modified silica gel glass plates (water/MeCN 9/1 *v*/*v*) and led to the desired compound as a white solid (24 mg). Final washes of the desalted product with acetonitrile (1 × 1 mL), then with acetone (1 × 1 mL) were necessary to remove residues of the modified silica absorbent (cyanopropyl and others) and led to the desired **GaF(L)** gallium complex as a white solid. Yield (extrapolated from the purified portion of crude material): 73% (21 mg, based on **GaF(L)** anhydrous form).

### For X = OH

An aqueous solution of 0.0529 *M* (ICP evaluation) gallium nitrate (4.9 mL, 0.259 mmol, 1 eq.) was added to the previously prepared **LH₂** ligand isolated at *pH 6* (150 mg, 0.261 mmol based on anhydrous mono-sodium form). The *pH* of the reaction solution dropped to *pH* 1.5. A 1 *M* and 2 *M* aqueous solution of NaOH was gradually added to adjust the *pH* to 4.7 (0.585 mmol, 2.2 eq.). The reaction was monitored by mass spectrometry and proved complete after less than 5 minutes at room temperature. The fine solid formed was filtered out over a Celite^{®} pad. The aqueous filtrate was then treated with Chelex^{®}100 resin (sodium form, 11 mg), stirred for 24 hours and centrifuged to collect the supernatant. This operation was repeated once. The solution was concentrated to dryness. The desalting process was further completed by chromatography on reverse phase chromatography on cyano(propyl)-modified silica gel (water/acetone 9/1 *v*/*v*) and led to the desired product as a white solid (91 mg, 55%).

### Example 8

### ¹⁸F Radiolabeling and stability study

### Preparation of the stock solutions

A stock solution in ultrapure water of **Ga(NO₃)₃** at concentration c 201.6 mM (ICP titration) was used. Stock solutions in ultrapure water of **LH₂, GaF(L),** or **GaOH(L)** were prepared at concentration *c* ≈ 200 m*M*. **AcONa/H** buffer solutions at *c* 0.1 *M* were prepared at *pH* 4.0 and *pH* 5.5.

### Fluorine-18 production

[¹⁸F]F⁻ was produced with ¹⁸O enriched water via the [¹⁸O(p,n)¹⁸F] nuclear reaction for 10-15 min in a cyclotron (Cyclone 18/9, IBA). 2.2 mL of [¹⁸F]F⁻ in water with an activity between 10 and 30 GBq arrived in the automated synthesizer (AllinOne, Trasis) for its purification and elution. The aqueous solution of [¹⁸F]F⁻ was loaded onto a QMA cartridge (Sep-Pak Accell Plus QMA Plus Light, Waters) equilibrated with ultrapure water. After the loading, sample was rinsing with 5 mL of ultrapure water to remove any dissolved impurities. [¹⁸F]F⁻ was then eluted with 2 mL of 0.9% NaCl in a vial that has been placed in a shield container beforehand. Radioactivity of the final solution [¹⁸F]NaF was measured with a dose calibrator (PET Dose 5 Ci, COMECER), activity concentration was approximately 10 GBq/mL. This solution was used as is, with the activity concentration naturally decayed in proportion to the time elapsed since production. For some assays, the [¹⁸F]NaF solution was diluted in 0.9% NaCl to reduce its activity concentration.

### Radiolabeling

### Method 1: One-pot radiolabeling

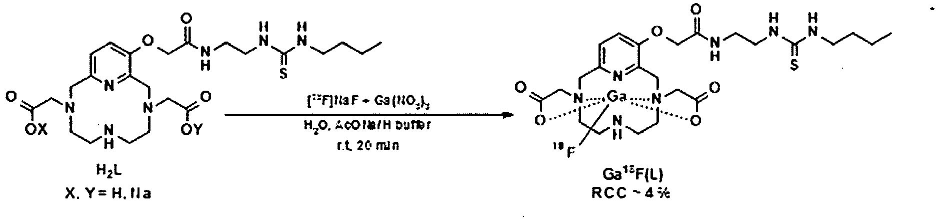

### Method 2: Radiolabeling via isotopic exchange¹⁹F/¹⁸F

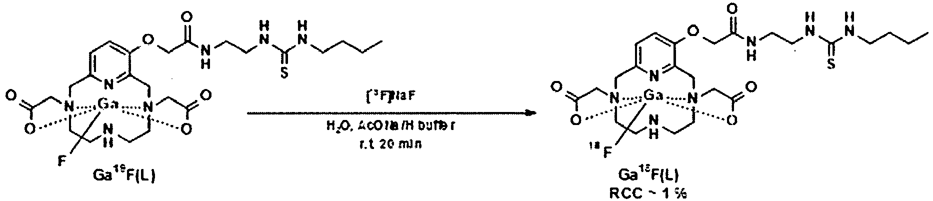

### Method 3: Radiolabeling via anionic exchange OH/¹⁸F

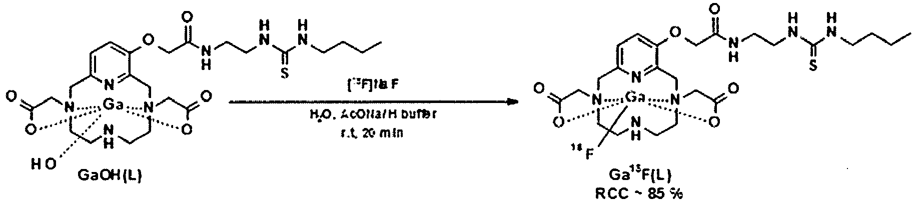

### • Method 1: One-pot with chelating agent.

Radiolabeling was performed by adding the chelating agent **LH₂** (30 µL of the stock solution, 6 µmol), **Ga(NO₃)₃** (15 µL of the stock solution, 3.0 µmol, 0.5 eq.), **AcONa/H** buffer (30 µL, pH 5.5, 0.1M) and **[¹⁸F]NaF** (50 µL, 271 ± 17 MBq, n = 3) in a glass test tube at room temperature for 20 min.

### • Method 2: Radiolabeling by isotopic exchange.

Radiolabeling was performed by adding **Ga¹⁹F(L)** (30 µL of the stock solution, 6 µmol), **AcONa/H** buffer (45 µL, *pH* 4.0, 0.1M) and **[¹⁸F]NaF** (50 µL, 120 MBq) in a glass test tube at room temperature for 20 min.

### • Method 3: Radiolabeling with preformed gallium complexes.

Radiolabeling was performed by adding **GaOH(L)** (30 µL of the stock solution, 6 µmol), **AcONa/H** buffer (45 µL, *pH* 4.0, 0.1*M*) and **[¹⁸F]NaF** (50 µL of a solution naturally decayed at 78 MBq.) in a glass test tube at room temperature for 20 min, resulting pH ≈ 5.2 (*pH* test strip).

### Determination of radiochemical conversion (RCC)

The radiochemical conversion (RCC) was determined by radio-HPLC operated on a ThermoFisher^{®} Dionex Ultimate 3000 HPLC system equipped with LPG-3400SD pump, WPS-3000SL/TSL autosampler, TCC-3000SD column oven, DAD-3000 detector and coupled with an Eckert Ziegler^{®} Mini-Scan TLC scanner and Flow-Count detector. Data were acquired with Chromeleon^{™} software (version 6.8; ThermoFisher^{®}). RCC was determined based on the relative percentage area of the peaks.

Analytical method: Column: Kinetex EVO C18 100 × 4.6 mm × 5 µm (Phenomenex^{®}) set at 25 °C. Eluent: Phase A: 5 *mM* AcONa/H buffer at *pH* 4.0. Phase B: acetonitrile. Gradient: A/B: 0-2 min: 95/5, 2-7 min: 95/5 to 70/30, 7-9 min: 70/30, 9-12 min: 70/30 to 40/60, 12 - 13 min : 40/60, 13 - 17 min: 40/60 to 95/5, 17 - 20 min: 95/5). Flow rate: 1 mL/min. The dead volume of the system was evaluated by injection of AcONa/H buffer: t_{R} = 0.95 min. Sample: crude reaction mixture; injection volume: 5 µL.

### Stability study of Ga¹⁸F(L)

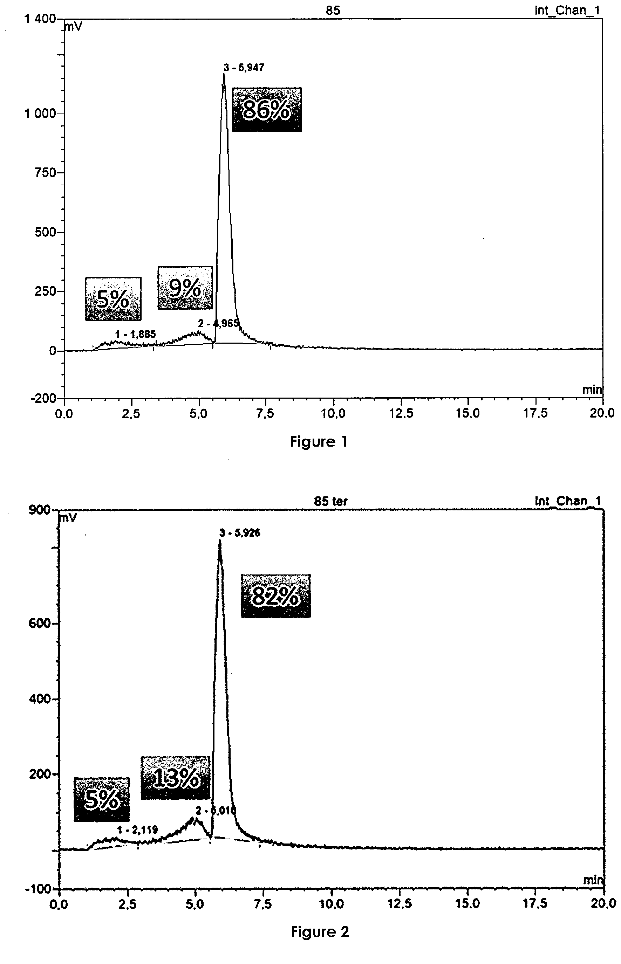

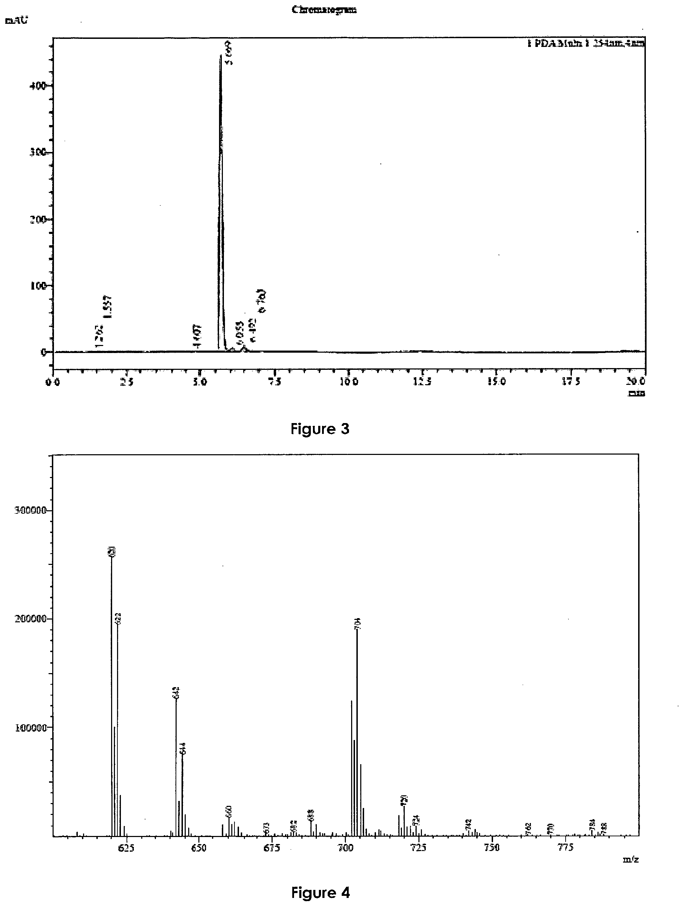

For this study, the assay with the highest RCC (radiolabeling from Method 3) was considered. 5 µL of the crude solution was injected into the radio-HPLC at several times measured from the introduction of **[¹⁸F]NaF:** 20 min ("*t₀*") and 60 min (see **Figures 1 and 2**). The LC-MS-UV analysis (see **Figures 3 and 4**) shows a peak at 5.7 min which corresponds to the complex **GaOH(L)** as fluorine-18 decays in oxygen-18.

## Claims

1. A complex of F, in particular ¹⁸F, and of a compound of the following formula (I): 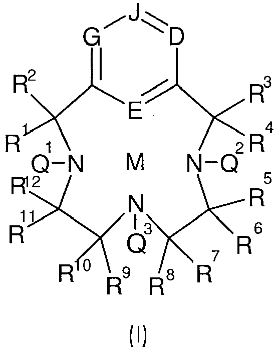 wherein:
- E represents CH, N or NO;
- M represents a cation selected from the list consisting of Al³⁺, Ga³⁺, In³⁺, Sc³⁺, Y³⁺, La³⁺, Ce³⁺, Pr³⁺, Nd³⁺, Sm³⁺, Eu³⁺, Gd³⁺, Tb³⁺, Dy³⁺, Ho³⁺, Er³⁺, Tm³⁺, Yb³⁺, Lu³⁺ and Fe³⁺, and is coordinated to at least one of the N atoms linked to Q¹, Q² and Q³ and to E when E represents N or NO;
- D, G and J, identical or different, represent a group selected from the list consisting of CH, N, C-OH, C-O-L and C-L, wherein L represents a linker group, optionally linked to a biological targeting moiety (BTM);
- R¹, R², R³, R⁴, R⁵, R⁶, R¹¹ and R¹², identical or different, represent a group selected from the list consisting of -H, -OH, -(CH₂)ₙ-CH₃, -(CH₂)ₙ-OH and -L, wherein n represents an integer from 0 to 5,
- R⁷, R⁸, R⁹ and R¹⁰, identical or different, represent a group selected from the list consisting of -H, =O, -(CH₂)ₚ-CH₃, -(CH₂)ₚ-OH and -L, wherein p represents an integer from 0 to 3, provided that when one of R⁷ or R⁸ and/or one of R⁹ or R¹⁰ represents =O then the other one is absent, i.e. represents no group;
- Q¹, Q² and Q³, identical or different, represent a group selected from the list consisting of -H, -OH, -(CH₂)ₖ-CH₃, -(CH₂)ₖ-OH, -CHQ⁴X and -L, wherein k represents an integer from 0 to 3, Q⁴ represents H or a C₁₋₃ alkyl group, and X represents a group selected from the list consisting of -COO-, -PO(OH) (O-) and -Q⁵-PO(OH), wherein Q⁵ represents Ar or a C₁₋₃ alkyl group, provided that at least two of Q¹, Q² and Q³ represent -CHQ⁴X and are coordinated to M; provided that the compound of formula (I) comprises at least one L,
wherein F, in particular ¹⁸F, is coordinated to M.

2. The complex according to claim 1, wherein the compound is of the following formula (I-1): 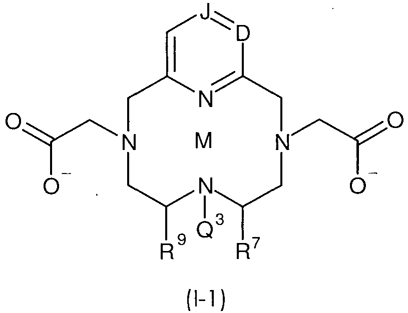 wherein:
- R⁷ and R⁹, identical or different, represents =O or -H, and
- D represents C-O-L, J represents C-H, and Q³ represents -H, -OH, -(CH₂)ₖ-CH₃, -(CH₂)ₖ-OH wherein k represents an integer from 0 to 3, or
- D represents C-H, J represents C-L or C-O-L, and Q³ represents -H, -OH, -(CH₂)ₖ-CH₃, - (CH₂)ₖ-OH wherein k represents an integer from 0 to 3, or
- D represents C-H, J represents C-H, and Q³ represents L,
wherein L is as defined in claim 1.

3. The complex according to claim 1 or 2, wherein L is a group of formula -(A)ₘ-Y wherein
- Y represents a group selected from the list consisting of -H, -N=C=S, -N=C=O, - NH(C=S)-BTM, and -NH(C=O)-BTM,
- each A is independently selected from the list consisting of -CR₂-, -CR=CR-, -C≡C-, - CR₂CO₂-, -CO₂CR₂-, -NRCO-, -CONR-, -CONHCR₂NH(C=S)NH-, -CONHCR₂NH(C=O)NH- , -CR=N-O-, -NR(C=O)NR-, -NR(C=S)NR-, -SO₂NR-, -NRSO₂-, -CR₂OCR₂-, -CR₂SCR₂-, - CR₂NRCR₂-, -CR₂NH(C=S)NH-, -CR₂NH(C=O)NH-, a C₄₋₈ cycloheteroalkylene group, a C₄₋₈ cycloalkylene group, -Ar-, -NR-Ar-, -O-Ar-, -Ar-(CO)-, an amino acid, a sugar or a polyethyleneglycol (PEG) building block,
wherein:
- each R is independently selected from the list consisting of H, C₁₋₆ alkyl, C₂₋₄ alkenyl, C₂₋₄ alkynyl, C₁₋₄ alkoxyalkyl, C₁₋₄ hydroxyalkyl, Ar-NH(C=S)NH, Ar-NH(C=O)NH, C₁₋₄ alkylphenyl-NH(C=S)NH, C₁₋₄ alkylphenyl-NH(C=O)NH,
- m is an integer from 1 to 20,
- each Ar is independently a C₅₋₁₂ arylene group, or a C₃₋₁₂ heteroarylene group.

4. The complex according to anyone of claims 1 to 3, wherein L is selected from the list consisting of:

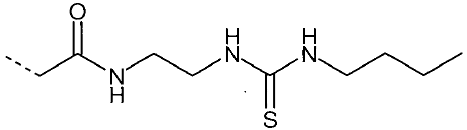

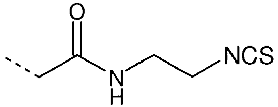

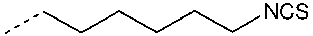

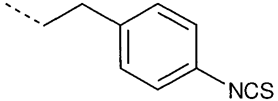

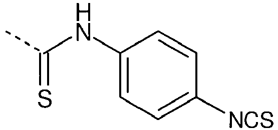

5. The complex according to anyone of claims 1 to 4, wherein M represents Ga³⁺.

6. The complex according to anyone 1 to 5, wherein the compound is represented by a formula selected from the list consisting of:

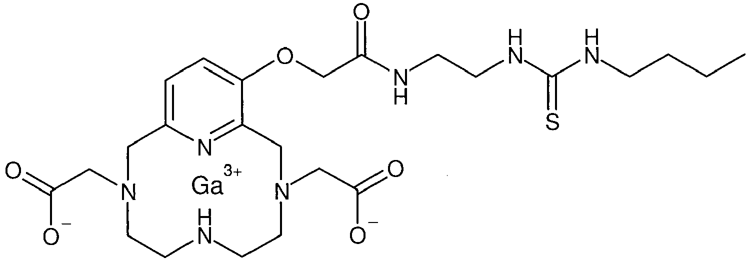

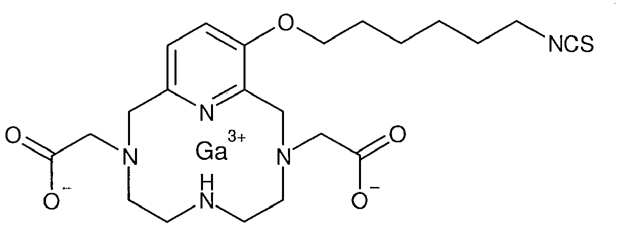

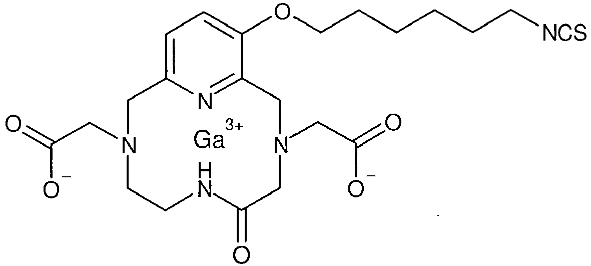

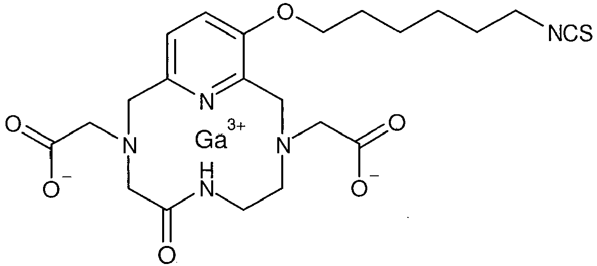

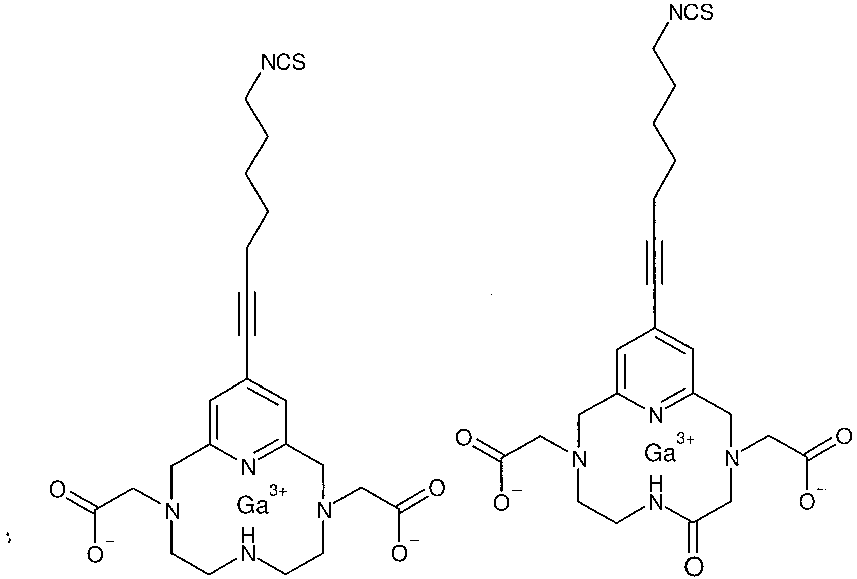

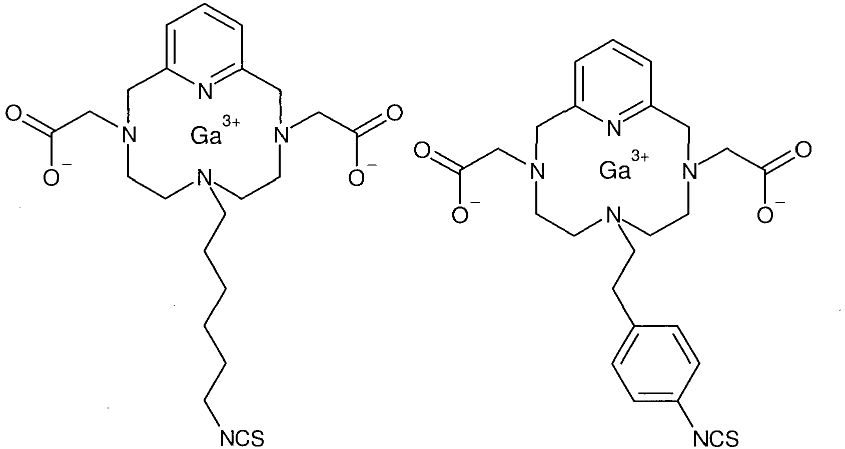

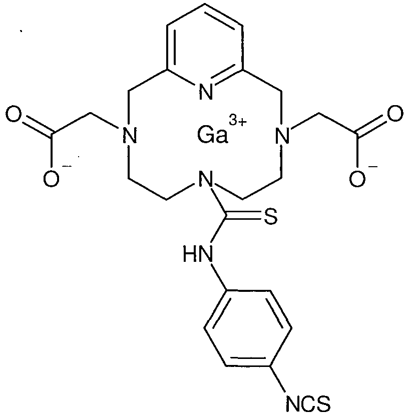

7. The complex according to any one of claims 1 to 6, which is linked to a biological targeting moiety (BTM).

8. The complex according to any one of claims 1 to 7, wherein the biological targeting moiety (BTM) is selected from the list consisting of a pre-targeting moiety, an amino-acid, a polypeptide, a peptoid or peptidic mimetic, an enzyme substrate, agonist or inhibitor, a ligand of a biological receptor, an antibody, an antibody fragment, a scFv, a VHH and an aptamer.

9. The complex according to any one of claims 1 to 8, wherein F is ¹⁸F and the compound is linked to a biological targeting moiety (BTM), for use as an *in vivo* PET scan diagnostic agent.

10. The use of a complex according to any one of claims 1 to 8, wherein F is ¹⁸F and the compound is linked to a biological targeting moiety (BTM), for generating a PET scan image of a body or a body part of an individual, wherein the complex has been administered previously to the individual.

11. A method of imaging an individual, comprising generating an image, using PET, of a body or body part of the individual, to which a complex as defined in any one of claims 1 to 8, wherein F is ¹⁸F and the compound is linked to a biological targeting moiety (BTM), has distributed, wherein the complex has been administered previously to the individual.

12. A pharmaceutical, imaging or diagnostic composition comprising a complex as defined in any one of claims 1 to 8, wherein F is ¹⁸F and the compound is linked to a biological targeting moiety (BTM), optionally in association with at least one biocompatible carrier.

13. A method of preparation of the complex according to any one of claims 1 to 8, which comprises:
- reacting a compound of formula (I), wherein M bound to R¹³, with a ¹⁸F fluoride anion, wherein R¹³ represents -OH, -OCH₃, -OCH₂-CH₃, -CO-CH₃, -CO-CH₂-CH₃, or
- reacting a complex of ¹⁹F and a compound of formula (I) wherein ¹⁹F is coordinated to M, with a ¹⁸F fluoride anion, or
- reacting a complex of ¹⁸F and M, wherein M is as previously defined, with a compound of the following formula (I'): 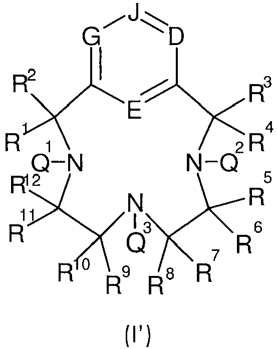
wherein D, E, G, J, Q¹, Q², Q³, R¹, R², R³, R⁴, R⁵, R⁶, R⁷, R⁸, R⁹, R¹⁰, R¹¹ and R¹² are as previously defined.

14. A kit or cassette comprising the non-radioactive reagents necessary to carry out the method of claim 13, including:
- a compound of formula (I), wherein M is bound to R¹³, wherein R¹³ represents -OH, - OCH₃, -OCH₂CH₃, -CO-CH₃, -CO-CH₂-CH₃, or
- a complex of ¹⁹F and a compound of formula (I) wherein ¹⁹F is coordinated to M, or
- a compound of formula (I').

15. A compound of formula (I) as defined in any one of claims 1 to 8, wherein M is bound to R¹³, wherein R¹³ represents -OH, -OCH₃, -OCH₂-CH₃, -CO-CH₃, or -CO-CH₂-CH₃, preferably -OH
